# EUROPÄISCHE PATENTSCHRIFT

(11) **EP 1 185 158 B1**
(45) Veröffentlichungstag und Bekanntmachung des Hinweises auf die Patenterteilung: **26.03.2003**
(21) Anmeldenummer: 00931205.9
(22) Anmeldetag: 12.05.2000
(51) Int. Cl.: A01D 43/00, C12P 21/00, C07K 14/415

(54) **VERFAHREN ZUM HERSTELLEN VON STRESSPROTEINEN AUS PFLANZLICHEM MATERIAL UND ERNTEMASCHINE ZUM DURCHFÜHREN DES VERFAHRENS**
METHOD FOR PRODUCING STRESS PROTEINS FROM VEGETABLE MATERIAL AND HARVESTING MACHINE FOR IMPLEMENTING SAID METHOD
PROCEDE DE PRODUCTION DE PROTEINES DU STRESS A PARTIR DE MATIERES VEGETALES ET MOISSONNEUSE POUR LA MISE EN OEUVRE DE CE PROCEDE

(30) Priorität: 19.05.1999 GE 348799
(43) Veröffentlichungstag der Anmeldung: 13.03.2002
(73) Patentinhaber: Dr. August Wolff GmbH & Co. Arzneimittel, 33611 Bielefeld (DE)
(72) Erfinder: JALIASHVILI, Tengiz, D-48529 Nordhorn (DE); JALIASHVILI, Nata, D-48529 Nordhorn (DE)
(74) Vertreter: Grünecker, Kinkeldey, Stockmair & Schwanhäusser Anwaltssozietät
(86) Internationale Anmeldenummer: EP0004313
(87) Internationale Veröffentlichungsnummer: WO00070931

(56) Entgegenhaltungen:
- FERULLO JEAN-MARC ET AL: "Post-harvest alteration of in vitro translatable mRNA population in alfalfa (Medicago sativa L.)." CROP SCIENCE, Bd. 36, Nr. 4, 1996, Seiten 1011-1016, XP002146910 ISSN: 0011-183X

## Beschreibung

Die Erfindung betrifft ein Verfahren zum Herstellen von Stressproteinen aus pflanzlichem Material und eine Erntemaschine zum Verwenden beim Verfahren.

Es ist bekannt, dass Pflanzen Stress-sensible Gene besitzen, die diesen Toleranz gegenüber der Veränderung von Umweltbedingungen verleihen. Diese Veränderung kann in einer Änderung der Temperatur, der Lichtbedingungen, der Wasserversorgung oder der hormonellen Umstände bestehen. Aufgrund dieser veränderten Umweltbedingungen werden die Stress-sensiblen Gene in Pflanzen exprimiert und die Synthese der meisten anderen Proteine unterdrückt. Beispielsweise zeigen Pflanzen, wenn sie normalerweise tödlichen Temperaturen ausgesetzt werden, Toleranz gegenüber diesen Temperaturen, wenn sie zuerst einer Behandlung bei hohen, jedoch nicht tödlichen, Temperaturen, unterworfen werden, wie in Annu. Rev. Plant Physiology Plant Mol. Biol. 1991, 42, Seiten 579 bis 620 beschrieben.

Der Mechanismus der Geninduktion von Stress-sensiblen Genen in Pflanzen durch Wärme und die dadurch exprimierten Stressproteine selbst sind in der Natur stark konserviert. Beispielsweise haben die meisten Arten von Stressproteinen, deren Produktion durch Wärme induziert wird, ein Molekulargewicht von 80 bis 90 kDa, 68 bis 74 kDa und 18 bis 30 kDa.

Die genaue Funktion von Stressproteinen ist derzeit nicht bekannt, wobei angenommen wird, dass diese die Zellen vor negativen Auswirkungen veränderter Umweltbedingungen schützen. Daher sind Verwendungen von Stressproteinen auf dem Gebiet der Medizin, Veterinarmedizin, Molekularbiologie, Pharmazie, Kosmetik und Pflanzenschutz bekannt.

Verfahren zum Charakterisieren und Isolieren geringer Mengen an Stressproteinen aus Pflanzen unter Verwendung von Gelelektrophorese sind bekannt.

In Plant Physiol., 1996, Seiten 1038 bis 1047 ist die Charakterisierung von cDNAs und Stressproteinen und die Expression dieser Stressproteine während eines Wärmeschocks von Pflanzen beschrieben. Dazu werden 8 bis 9 Tage alte Pflanzen einem Wärmeschock ausgesetzt, indem die Temperatur des Wachstumsraums mit einer Geschwindigkeit von 4°C pro Stunde erhöht wird, bis die erwünschte Temperatur von 30 bis 40°C erreicht ist. Diese Temperatur wird 4 Stunden beibehalten und sodann wird mit einer Geschwindigkeit von 4°C pro Stunde abgekühlt, bis eine Temperatur von ca. 22°C erreicht ist. Die Stressproteine werden aus dem so erhaltenen pflanzlichen Material mit Elektrophorese isoliert.

In Plant Physiol., 1993, 101, Seiten 1209 bis 1216 wird eine Untersuchung der Expression von Stressproteinen in Pflanzen beschrieben. Dazu werden wiederum die Pflanzen einem Wärmeschock ausgesetzt, indem diese einem Erhöhen der Temperatur mit einer Geschwindigkeit von 4°C pro Stunde und 4 Stunden Behandein bei der Maximaltemperatur ausgesetzt werden. Sodann wurden Proben der Blätter dieser Pflanzen für die Proteinanalyse genommen. Diese Proben wurden in Plastiksäcke eingebracht und auf Eis gelagert, bis diese mit Gelelektrophorese zur Isolierung der Stressproteine weiterverarbeitet werden.

Außerdem sind Verfahren zur Herstellung von Stressproteinen mittels Gentechnologie bekannt.

Diese Verfahren haben den Nachteil, dass sie aufwendig und teuer sind sowie nur geringe Mengen an Stressproteinen liefern. Wegen der vielfältigen Anwendungsmöglichkeiten von Stressproteinen in der Industrie, besteht daher ein Bedarf an einem Verfahren zur großtechnischer Herstellung von Stressproteinen.

Der vorliegenden Erfindung liegt daher die Aufgabe zugrunde, ein Verfahren zur Verfügung zu stellen, das einfach und kostengünstig durchführbar ist, und eine größere Menge an Stressproteinen liefert.

Die Lösung dieser Aufgabe ist ein Verfahren zum Herstellen von Stressproteinen aus pflanzlichem Material, die Schritte umfassend:
(a) Ernten der Pflanzen mittels einer Erntemaschine, wodurch gemähtes, pflanzliches Material erhalten wird,
(b) Erwärmen des pflanzlichen Materials zur Induktion der Produktion von Stressproteinen in diesem pflanzlichen Material unmittelbar vor und/oder während und/oder nach Schritt (a), und
(c) Isolieren der Stressproteine aus dem pflanzlichen Material.

Eine weitere der Erfindung zugrunde liegende Aufgabe ist es, eine Erntemaschine zum Verwenden beim erfindungsgemäßen Verfahren zur Verfügung zu stellen.

Eine Lösung dieser Aufgabe ist eine Erntemaschine zum Verwenden beim erfindungsgemäßen Verfahren, umfassend ein motorgetriebenes Fahrzeug und eine an dem Fahrzeug gelagerte Mähvorrichtung, wobei an der Mähvorrichtung und/oder dem Fahrzeug eine auf die Pflanzen vor der Mähvorrichtung und/oder auf den Bereich der Mähwerkzeuge gerichtete Wärmequelle vorgesehen ist.

Eine weitere Lösung dieser Aufgabe ist eine Erntemaschine zum Verwenden beim erfindungsgemäßen Verfahren, umfassend ein motorgetriebenes Fahrzeug und eine an dem Fahrzeug gelagerte Mähvorrichtung, wobei eine vom Fahrzeug bewegte oder selbstfahrende Sammelvorrichtung mit einem Förderkanal und einem Sammelbehälter zum Aufnehmen und Transportieren des pflanzlichen Materials vorgesehen ist, wobei der Förderkanal und/oder der Sammelbehälter so ausgestaltet sind, dass im pflanzlichen Material ein Erwärmen aufgrund endo-biochemischer Vorgänge stattfindet.

Eine andere Lösung dieser Aufgabe ist die Kombination der vorstehend genannten Emtemaschinen.

In den Zeichnungen sind Ausführungsbeispiele einer erfindungsgemäßen Emtemaschine dargestellt. Es zeigen:
Fig. 1 eine Seitenansicht einer ersten Ausführungsform;
Fig. 2 eine Draufsicht auf die Darstellung nach Fig. 1;
Fig. 3 eine Teildarstellung eines zweiten Ausführungsbeispiels mit einer auf die Pflanzen vor der Mähvorrichtung gerichteten Wärmequelle in Form eines Gebläses; und
Fig. 4 ein Diagramm, in dem die Abhängigkeit der Stressprotein-Induktion und -Produktion von der Temperatur dargestellt ist.

Im Sinne der vorliegenden Erfindung umfasst der Begriff "pflanzliches Material" sowohl Pflanzen als auch gemähtes, pflanzliches Material. Im Sinne der vorliegenden Erfindung bedeutet der Begriff "Stressproteine" diejenigen Proteine, die in pflanzlichem Material durch dessen Erwärmung hergestellt werden.

Das Ernten der Pflanzen in Schritt (a) umfasst das Schneiden der Pflanzen mit rotierenden oder oszillierenden Schneidwerkzeugen oder das Abschlagen mit Schlagwerkzeugen.

Die in dem erfindungsgemäßen Verfahren verwendbaren Pflanzen können vorzugsweise ausgewählt werden aus Alfalfa, Sojabohnen, Gras, Getreide, Algen und Wasserpflanzen.

Durch das Erwärmen des pflanzlichen Materials in Schritt (b) unmittelbar und/oder während und/oder nach Schritt (a) wird die Produktion von Stressproteinen in dem pflanzlichen Material induziert. Das Erwärmen des pflanzlichen Materials in Schritt (b) des erfindungsgemäßen Verfahrens kann sowohl vor, als auch während sowie nach dem Schritt (a) durchgeführt werden, um die Produktion an Stressproteinen im pflanzlichen Material während des gesamten Emtens und danach zu gewährleisten.

Vorzugsweise wird jedoch das pflanzliche Material nach dem Schritt (a) erwärmt, um das erfindungsgemäße Verfahren zu vereinfachen.

Insbesondere bevorzugt wird das Erwärmen nach Schritt (a) des erfindungsgemäßen Verfahrens zumindest zum Teil oder gänzlich durch Erwärmen auf Grund endo-biochemischer Vorgänge im pflanzlichen Material durchgeführt. Dadurch ist das erfindungsgemäße Verfahren einfach und umweltfreundlich durchführbar, da keine Fremdenergie für das Erwärmen benötigt wird.

Das Erwärmen kann dadurch bewirkt werden, dass das pflanzliche Material unmittelbar nach dem Schritt (a), vorzugsweise innerhalb etwa 1 Stunde, insbesondere innerhalb etwa 30 Minuten, am bevorzugtesten innerhalb 1 Minute, in im wesentlichen luftdichte Behälter eingebracht wird. Die Temperatur des pflanzlichen Materials in den verschlossenen Behältern steigt nach dem Verschließen aufgrund anaerober, endo-biochemischer Vorgänge im pflanzlichen Material mit einer Geschwindigkeit von etwa 5°C pro Stunde an. Die Temperatur kann dabei bis zu maximal etwa 60°C erreichen. Dadurch wird das pflanzliche Material einem Warmeschock unterworfen und die Induktion der Produktion von Stressproteinen in dem pflanzlichen Material bewirkt.

Das Erwärmen des pflanzlichen Materials unmittelbar vor und/oder während Schritt (a) des erfindungsgemäßen Verfahrens wird vorzugsweise unter Verwendung eines Strahlers und/oder Gebläses bewirkt. Der Strahler kann ein Mikrowellenstrahler sein.

Das Erwärmen des pflanzlichen Materials in Schritt (b) des erfindungsgemäßen Verfahrens wird vorzugsweise auf eine Temperatur im Bereich von etwa 30 bis etwa 60°C durchgeführt.

Das Erwärmen des pflanzlichen Materials unmittelbar vor Schritt (a) des erfindungsgemäßen Verfahrens wird bevorzugt auf eine Temperatur im Bereich von etwa 50 bis etwa 60°C, insbesondere auf etwa 55°C, durchgeführt. Das Erwärmen während und/oder nach Schritt (a) wird bevorzugt auf eine Temperatur im Bereich von etwa 35 bis etwa 45°C, insbesondere etwa 36 bis etwa 40°C, am bevorzugtesten etwa 38 bis 40°C durchgeführt.

Das Erwärmen des pflanzlichen Materials in Schritt (b) des erfindungsgemäßen Verfahrens wird vorzugsweise bis zu etwa 75 Stunden durchgeführt.

Wenn das Erwärmen des pflanzlichen Materials in Schritt (b) des erfindungsgemäßen Verfahrens unmittelbar vor Schritt (a) durchgeführt wird, ist es bevorzugt das Erwärmen für einen Zeitraum von etwa 1 Sekunde bis etwa 1 Minute, insbesondere für etwa 30 Sekunden, durchzuführen. Wenn das Erwärmen in Schritt (b) des erfindungsgemäßen Verfahrens nach Schritt (a) durchgeführt wird, ist es bevorzugt, das Erwärmen bis zu etwa 75 Stunden, insbesondere bis zu 72 Stunden, bevorzugter etwa 2 bis etwa 6 Stunden und am bevorzugtesten etwa 3 bis etwa 5 Stunden durchzuführen.

Das pflanzliche Material kann vor dem Erwärmen nach dem Schritt (a) zerkleinert werden, es ist jedoch bevorzugt, das pflanzliche Material vor dem Erwärmen nach dem Schritt (a) nicht zu zerkleinern, um eine optimale Produktion von Stressproteinen während des Erwärmens zu gewährleisten.

Vorzugsweise kann das pflanzliche Material zum Zwecke des Erwärmens aufgrund endo-biomechanischer Vorgänge nach dem Schritt (a) verdichtet werden.

Das Erwärmen in Schritt (b) des erfindungsgemäßen Verfahrens kann vorzugsweise zumindest teilweise unter Verwendung der Abwärme des Antriebs der in Schritt (a) verwendeten Erntemaschine durchgeführt werden, wodurch der Energieverbrauch beim Durchführen des erfindungsgemäßen Verfahrens vermindert wird.

Aus dem nach Schritt (b) erhaltenen pflanzlichen Material werden sodann in Schritt (c) des erfindungsgemäßen Verfahrens die Stressproteine isoliert. Die Isolierung wird mit herkömmlichen Verfahren durchgeführt. Beispielsweise wird das in Schritt (b) erhaltene pflanzliche Material, gegebenenfalls nach Zerkleinern, in einer horizontalen Presse gepresst. Dadurch wird ein Presskuchen und der Saft der Zellen des pflanzlichen Materials erhalten. Der Saft der Zellen wird einer thermischen Behandlung unterworfen und sodann zentrifugiert. Dadurch wird ein proteinhaltiger Überstand und unlösliches Materials erhalten. Der Überstand wird mittels Cross-Flow-Filtration konzentriert, wodurch ein Konzentrat erhalten wird, das die Stressproteine enthält. Die Stressproteine werden aus diesem Konzentrat mit herkömmlichen Verfahren isoliert, wie durch Fällung mit Aceton oder Vakuumtrocknen. Dadurch werden die in dem pflanzlichen Material enthaltenen Stressproteine erhalten.

Das erfindungsgemäße Verfahren wird nachstehend anhand von Beispielen erläutert.

### Beispiel 1

400 kg Alfalfa wurden am frühen Morgen (6 Uhr morgens) geemtet, wenn dessen Temperatur etwa 8 bis 10°C betrug, um jede Art von Temperaturstress auszuschließen. Sodann wurde das so erhaltene pflanzliche Material sofort geschnitten und in vier 50 kg-Säcke eingebracht Die Temperatur des pflanzlichen Materials wurde fortlaufend in jedem Sack mit Thermometern uberwacht und alle 30 Minuten gemessen. Als Kontrollexperiment wurden 50 kg des pflanzlicnen Materials offen auf den Boden gelegt. In dem Kontrollexperiment war die Anderung der Temperatur des pflanzlichen Materials sehr gering und der Verlust an Zellsaft war merklich (20-30%). In den Säcken stieg die Temperatur des pflanzlichen Matenals allmahlich aufgrund endo-biochemischer Vorgänge an (+1,45°C/h) bis ca. 60°C erreicht waren

Die erwünschte Temperatur fur die induktion der Produktion von Stressproteinen von 38 bis 40°C in den vier, Sacken mit dem pflanzlichen Material wurde nach etwa 30 bis 34 Stunden nach dem Emten erreicht

### Beispiel 2

400 g Alfalfa wurden am frunen Morgen bei einer Umgebungstemperatur von 8 bis 10°C geerntet und in vier 50 kg Sacke eingebracht Die Temperatur in jedem Sack stieg mit einer Geschwindigkeit von 5.3°C pro Stunde an und die erwunschte Temperatur von 36 bis 40°C zum Ausüben des Warmeschocks wurde nach ca. 3 bis 5 Stunden nach dem Ernten erreicht. Nach 6 Stunden stieg die Temperatur des biologischen Materials nicht weiter an und verblieb bei 42 bis 45°C uber 72 Stunden und sank dann langsam ab.

Zur selben Zeit wurden als Kontrollexpenment 50 kg nicht geschnittenes Alfalfa auf den Boden gelegt, und es wurde festgestellt, dass der Temperaturanstieg während derselben 20 Stunden, wie vorstehend, nicht moglich war

### Beispiel 3

Frisch geschnittenes intaktes Alfalfa wurde gesammelt und in Säcke, wie in Beispiel 1 beschrieben, eingebracht. Wenn die Temperatur in den Säcken 28 bis 30°C, 37 bis 39°C oder 42 bis 45°C erreicht hatte, wurden Proben des Alfalfas mit einem Feuchtigkeitsgehalt von 78-82% genommen, in 3 bis 5 cm lange Stücke geschnitten, und sodann sofort in einer horizontalen Presse gepresst. Nach dieser mechanischen Extraktion des Zellsaft aus der gesamten Biomasse von Alfalfa wurden ein Presskuchen, der 60% der gesamten Biomasse mit einem Feuchtigkeitsgehalt von etwa 60% enthielt, und der Saft der Alfalfazellen, der 40% der gesamten Biomasse mit etwa 10 bis 12% Feststoffen enthielt, erhalten.

Der Presskuchen wurde mit 100 g Lactobacilli konserviert.

Der Saft der Alfalfazellen wurde 15 Minuten lang bei 95°C wärmebehandelt und sodann durch Zentrifugieren getrennt. Die Feststoffe wurden vom Überstand getrennt, wobei angenommen wird, dass die Feststoffe thermolabile Proteine waren. Der Überstand wurde 30fach mittels Cross-Flow-Filtration (NF45PPDSS) konzentriert. Das Konzentrat enthielt die thermostabilen Stressproteine und deren Zusammensetzung wurde mit eindimensionaler SDS-Gelelektrophorese bestimmt.

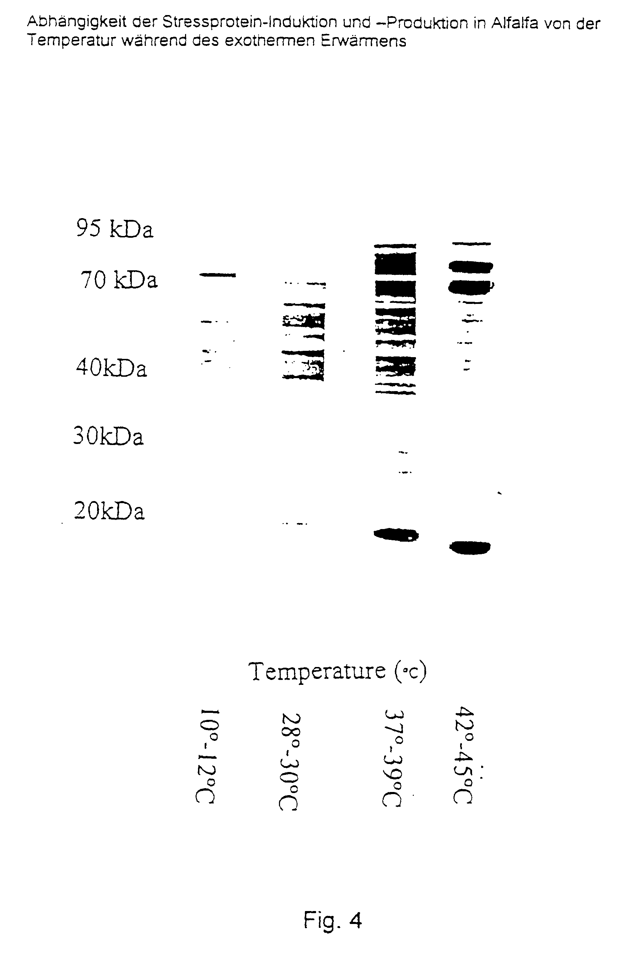

Die Ergebnisse der eindimensionalen SDS-Gelelektrophorese sind in Figur 4 gezeigt.

Der Fig. 4 ist zu entnehmen, dass der Gehalt an Stressproteinen bei einer Temperatur von 10 bis 12°C in Alfalfazellen vernachlässigbar ist. Wenn die Temperatur auf einen Bereich von 28 bis 30°C ansteigt, steigt auch die Produktion der Stressproteine signifikant an, und erreicht ein Maximum bei 37 bis 39°C. Ein weiterer Anstieg der Temperatur auf 42 bis 45°C vermindert wiederum die Anzahl an verschiedenen Stressproteinen. Außerdem bleibt dieselbe Anzahl an Stressproteinen in Alfalfa bis zu 20 Stunden bei Erwärmen aufgrund endo-biochemischer Vorgänge erhalten.

Die Gesamtausbeute an Stressproteinen ist nicht geringer als 30 bis 40 g aus einer Tonne frisch geerntetem Alfalfa.

Das erfindungsgemäße Verfahren dauert insgesamt etwa 6 bis 8 h.

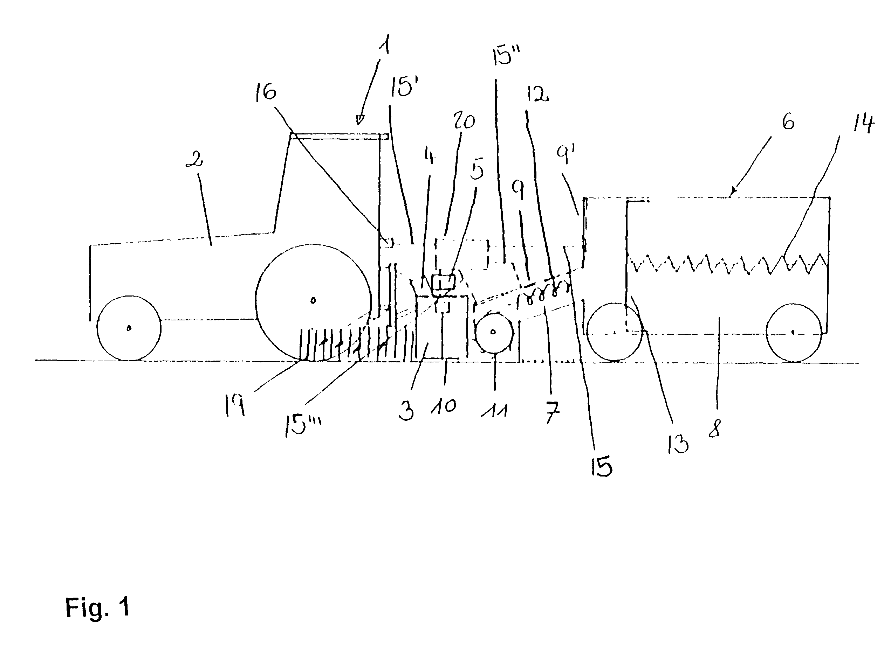

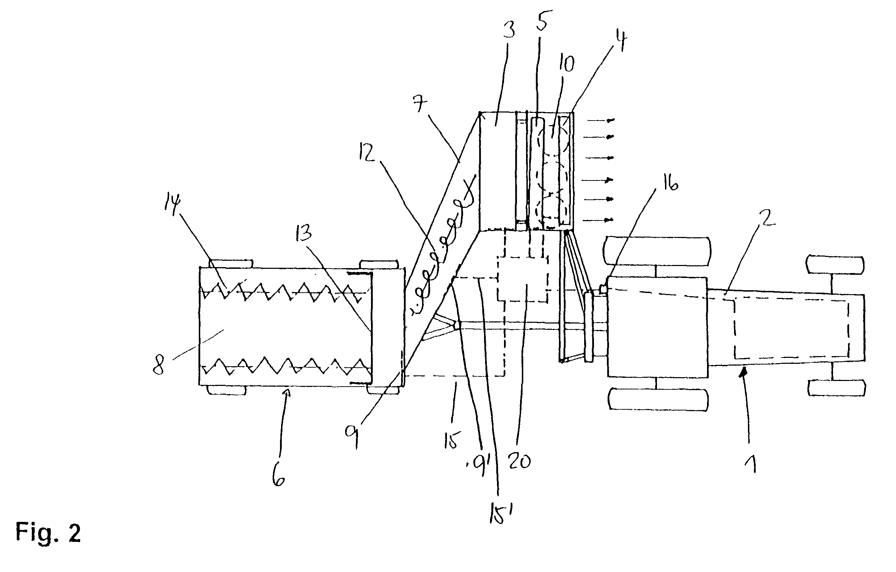

Das erfindungsgemäße Verfahren wird vorzugsweise mit Hilfe einer erfindungsgemäßen Erntemaschine 1, wie in Fig. 1 und 2 gezeigt, durchgeführt. Die Erntemaschine 1 umfasst ein motorgetriebenes Fahrzeug 2, insbesondere einen Traktor, und eine an dem Fahrzeug gelagerte Mähvorrichtung 3 mit Mähwerkzeugen 10, die im vorliegenden Fall als rotierende Mähmesser ausgebildet sind. Die Mähvorrichtung 3 ist mit einer auf die noch stehenden Pflanzen 19 vor der Mähvorrichtung gerichteten Wärmequelle 4 versehen, die ein Strahler, z. B. ein Infrarot- oder ein Mikrowellenstrahler, sein kann.

Die Mähvorrichtung 3 ist ferner mit einer auf den Bereich der Mähwerkzeuge 10 gerichteten Wärmequelle 5 versehen, über die Heißgas auf das Mähgut im Moment des Mähens gerichtet werden kann. Die Haube 5 kann auch durch einen Strahler ersetzt sein.

Die Erntemaschine 1 umfasst ferner eine vom Fahrzeug 2 bewegte oder selbstfahrende Sammelvorrichtung 6 mit einem Sammelbehälter 8 zum Aufnehmen und Transportieren des pflanzlichen Materials. Mit dem Sammelbehälter 8 ist ein Förderkanal 7 verbunden, an dessen vorderen Ende eine Pick-up-Walze 11 zum Aufnehmen des von Mähwerkzeugen 10 abgegebenen Erntegut angeordnet ist. In dem Förderkanal 7 befindet sich eine Förderschnecke 12 zum Fördern und Verdichten des Ernteguts. im Sammelbehälter 8 befindet sich eine Preßplatte 13, die durch einen Krafterzeuger 14 nach vorne zu elastisch belastet ist. Der Sammelvorrichtung 6 sind Wärmequellen 9, 9' zugeordnet, die im vorliegenden Fall als Röhren ausgebildet sind, durch die das Abgas des Antriebsmotors des Fahrzeugs 2 geleitet werden kann. Hierzu sind an dem Aufpuff 16 Kanäle 15, 15', 15" und 15"' sowie ein Verteiler 20 angeschlossen. Von dem Verteiler 20 aus führt eine Leitung zu der Haube 5. Der Sammelbehälter 8 und/oder der Förderkanal 7 der Sammelvorrichtung 6 können wärmeisoliert und/oder abgedichtet sein.

Beim Durchführen des erfindungsgemäßen Verfahrens mittels der Erntemaschine 1 werden die Pflanzen 19 mittels der Mähwerkzeuge 10 in der Mähvorrichtung 3 geschnitten, über die Pick-up-Walze 11 in den Förderkanal 7 eingebracht und mittels der Förderschnecke 12 in den Sammelbehälter 8 transportiert. Im Förderkanal und im Sammelbehälter 8 wird das pflanzliche Material verdichtet. Das pflanzliche Material erwärmt sich dabei und wird zusätzlich mittels der Wärmequelle 4 und/oder 5 und/oder 9, 9' erwärmt, je nachdem, welche dieser Wärmequellen vorgesehen bzw. aktiviert sind.

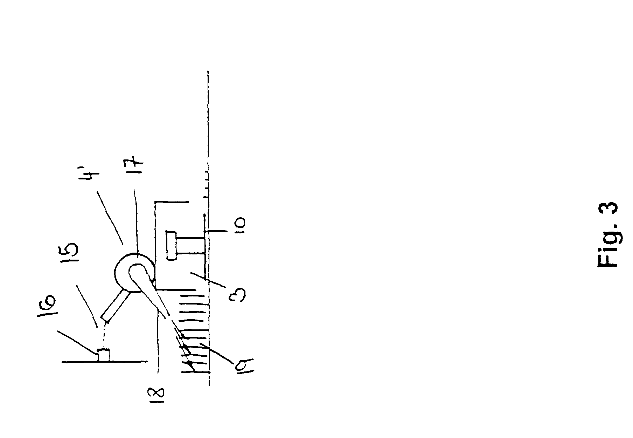

Bei der Ausgestaltung nach Fig. 3 ist die auf die Pflanzen 19 vor der Mähvorrichtung 3 gerichtete Wärmequelle 4' als Gebläse 17 ausgestaltet. Das Abgas des motorgetriebenen Fahrzeugs 2 wird dabei aus dem Auspuff 16 über ein Kanalsystem 15 dem Gebläse 17 zugeleitet. Aus dem Gebläse 17 wird über eine Leithaube, Düse 18 oder eine Leitfläche das heiße Abgas auf die Pflanzen 19 geleitet.

Das erfindungsgemäße Verfahren hat den Vorteil, einfach und kostengünstig durchführbar zu sein und große Mengen an Stressproteinen zu liefern. Außerdem sind die mit dem erfindungsgemäßen Verfahren hergestellten Stressproteine post-translational modifiziert und zeigen daher alle Funktionen, die bei der Exprimierung in Pflanzenzellen erhalten werden, im Gegensatz zu Stressproteinen, die chemisch oder in Bakterien hergestellt wurden. Außerdem hat das erfindungsgemäße Verfahren den Vorteil, wenn das Erwärmen durch endo-biochemische Vorgänge im pflanzlichen Material durchgeführt wird, ohne Verbrauch von Energie zum Erwärmen durchgeführt werden zu können, wodurch es umweltfreundlich ist. Dasselbe gilt, wenn das Erwärmen des pflanzlichen Materials mittels der Abwärme des motorgetriebenen Fahrzeugs 2 der Erntemaschine 1 durchgeführt wird.

## Patentansprüche

1. Verfahren zum Herstellen von Stressproteinen aus pflanzlichem Material, die Schritte umfassend:
(a) Ernten der Pflanzen mittels einer Erntemaschine, wodurch gemähtes, pflanzliches Material erhalten wird,
(b) Erwärmen des pflanzlichen Materials zur Induktion der Produktion von Stressproteinen in diesem pflanzlichen Material unmittelbar vor und/oder während und/oder nach Schritt (a), und
(c) Isolieren der Stressproteine aus dem pflanzlichen Material.

2. Verfahren nach Anspruch 1, wobei in Schritt (b) das Erwärmen nach Schritt (a) zumindest zum Teil durch Erwärmen aufgrund endo-biochemischer Vorgänge im pflanzlichen Material durchgeführt wird.

3. Verfahren nach einem der vorstehenden Ansprüche, wobei das Erwärmen auf eine Temperatur im Bereich von etwa 30 bis etwa 60 C° durchgeführt wird.

4. Verfahren nach Anspruch 3, wobei das Erwärmen unmittelbar vor Schritt (a) auf eine Temperatur von etwa 55 °C durchgeführt wird.

5. Verfahren nach Anspruch 3, wobei das Erwärmen während und/oder nach Schritt (a) auf eine Temperatur im Bereich von etwa 35 bis etwa 45 °C durchgeführt wird.

6. Verfahren nach einem der vorstehenden Ansprüche, wobei das Erwärmen bis zu etwa 75 h durchgeführt wird.

7. Verfahren nach Anspruch 6, wobei das Erwärmen unmittelbar vor Schritt (a) für einen Zeitraum von etwa 1 sec. bis etwa 1 min., insbesondere für etwa 30 sec., durchgeführt wird.

8. Verfahren nach Anspruch 6, wobei das Erwärmen nach Schritt (a) bis zu etwa 75 h durchgeführt wird.

9. Verfahren nach einem der vorstehenden Ansprüche, wobei das Erwärmen mindestens teilweise unter Verwendung der Abwärme des Antriebs der Erntemaschine durchgeführt wird.

10. Verfahren nach einem der vorstehenden Ansprüche, wobei das pflanzliche Material nach Schritt (b) zerkleinert wird.

11. Verfahren nach einem der vorstehenden Ansprüche, wobei das pflanzliche Material verdichtet wird.

12. Erntemaschine (1) zum Verwenden beim Verfahren nach einem der vorstehenden Ansprüche, umfassend ein motorgetriebenes Fahrzeug (2) und eine an dem Fahrzeug gelagerte Mahvornchtung (3), wobei an der Mähvorrichtung (3) und/oder dem Fahrzeug (2) eine auf die Pflanzen vor der Mähvorrichtung und/oder eine auf den Bereich der Mähwerkzeuge gerichtete Wärmequelle (4,5) vorgesehen ist.

13. Erntemaschine (1) zum Verwenden beim Verfahren nach einem der Ansprüche 1 bis 11, umfassend ein motorgetriebenes Fahrzeug (2) und eine an dem Fahrzeug gelagerte Mähvorrichtung (3), wobei eine vom Fahrzeug (2) bewegte oder selbstfahrende Sammelvomchtung (6) mit einem Förderkanal (7) und einem Sammelbehälter (8) zum Aufnehmen und Transportieren des pflanzlichen Materials vorgesehen ist, wobei der Forderkanal (7) und/oder der Sammelbehälter (8) so ausgestaltet sind, daß im pflanzlichen Material ein exothermes Erwärmen aufgrund endo-biochemischer Vorgänge stattfindet.

14. Erntemaschine (1) ausgestaltet nach den Ansprüchen 12 und 13.

15. Erntemaschine (1) nach einem der Ansprüche Anspruch 12 oder 13, wobei dem Förderkanal (7) und/oder dem Sammelbehälter (8) zugeordnete Wärmequelle (9) vorgesehen ist.

16. Erntemaschine (1) nach einem der Ansprüche 12, 14 oder 15, wobei die auf die Pflanzen vor der Mähvorrichtung und/oder auf die Mähwerkzeuge gerichtete Wärmequelle (4,5) als Strahler, Gebläse oder Mikrowellenstrahler (17) ausgebildet sind.

17. Erntemaschine (1) nach einem der Ansprüche 12 bis 15, wobei die Wärmequelle (4,5,9) mit der Abwärme des Fahrzeugs speisbar ist.

18. Erntemaschine (1) nach einem der Ansprüche 13 bis 17, wobei das Sammelfahrzeug (6) einen Förderkanal (7) und/oder einen Sammelbehälter (8) für das pflanzliche Material aufweist, die derart ausgestaltet sind, dass das pflanzliche Material in ihnen verdichtbar ist.

19. Erntemaschine (1) nach einem der Ansprüche 15 bis 18, wobei die dem Sammelfahrzeug (6) zugeordnete Wärmequelle (9) dem Förderkanal (7) und/oder dem Sammelbehälter (8) zugeordnet ist.

20. Erntemaschine (1) nach einem der Ansprüche 14 bis 19, wobei die dem Sammelfahrzeug (6) zugeordnete Wärmequelle (9) ein Kanalsystem (15) aufweist, durch das das Abgas des Fahrzeugs (2) leitbar ist.

21. Erntemaschine (1) nach einem der Ansprüche 13 bis 20, wobei der Förderkanal (7) und/oder der Sammelbehälter (8) wärmeisoliert sind.

22. Erntemaschine (1) nach einem der Ansprüche 13 bis 21, wobei der Förderkanal (7) und/oder der Sammelbehälter (8) abgedichtet sind.

## Claims

1. Method for producing stress proteins from vegetable material, comprising the following steps:
(a) harvesting the plants by means of a harvesting machine, whereby mown, vegetable material is obtained,
(b) heating the vegetable material to induce the production of stress proteins in this vegetable material immediately before and/or during and/or after step (a), and
(c) isolating the stress proteins from the vegetable material.

2. Method according to Claim 1, wherein the heating after step (a) is carried out in step (b) at least partly through heating on the basis of endobiochemical processes in the vegetable material.

3. Method according to either of the preceding Claims, wherein the heating is carried out to a temperature in the range from approximately 30 to approximately 60°C.

4. Method according to Claim 3, wherein the heating is carried out immediately before step (a) to a temperature of approximately 55°C.

5. Method according to Claim 3, wherein the heating is carried out during and/or after step (a) to a temperature in the range from approximately 35 to approximately 45°C.

6. Method according to any one of the preceding Claims, wherein the heating is carried out for up to approximately 75 h.

7. Method according to Claim 6, wherein the heating is carried out immediately before step (a) for a period of approximately 1 sec. to approximately 1 min., in particular for approximately 30 sec.

8. Method according to Claim 6, wherein the heating is carried out after step (a) for up to approximately 75 h.

9. Method according to any one of the preceding Claims, wherein the heating is carried out at least partly using the waste heat of the drive of the harvesting machine.

10. Method according to any one of the preceding Claims, wherein the vegetable material is comminuted after step (b).

11. Method according to any one of the preceding Claims, wherein the vegetable material is compressed.

12. Harvesting machine (1) for use in the method according to any one of the preceding Claims, comprising a motor-driven vehicle (2) and a mowing device (3) mounted on the vehicle, wherein a heat source (4, 5), which is directed at the plants before the mowing device and/or at the region of the mowing tools, is mounted on the mowing device (3) and/or the vehicle (2).

13. Harvesting machine (1) for use in the method according to any one of Claims 1 to 11, comprising a motor-driven vehicle (2) and a mowing device (3) mounted on the vehicle, wherein a collecting device (6), which is either moved by the vehicle or automotive, is provided, this having a delivery duct (7) and a collecting container (8) for taking up and transporting the vegetable material, wherein the delivery duct (7) and/or the collecting container (8) is/are designed such that exothermic heating on the basis of endobiochemical processes takes place in the vegetable material.

14. Harvesting machine (1) designed according to Claims 12 and 13.

15. Harvesting machine (1) according to either of Claims 12 and 13, wherein a heat source (9), which is associated with the delivery duct (7) and/or the collecting container (8), is provided.

16. Harvesting machine (1) according to any one of Claims 12, 14 or 15, wherein the heat source (4, 5) directed at the plants before the mowing device and/or at the mowing tools is formed as a radiator, blower or microwave radiator (17).

17. Harvesting machine (1) according to any one of Claims 12 to 15, wherein the heat source (4, 5, 9) can be fed with the waste heat of the vehicle.

18. Harvesting machine (1) according to any one of Claims 13 to 17, wherein the collecting vehicle (6) comprises a delivery duct (7) and/or a collecting container (8) for the vegetable material, these being designed such that the vegetable material can be compressed in them.

19. Harvesting machine (1) according to any one of Claims 15 to 18, wherein the heat source (9) which is associated with the collecting vehicle (6) is associated with the delivery duct (7) and/or the collecting container (8).

20. Harvesting machine (1) according to any one of Claims 14 to 19, wherein the heat source (9) which is associated with the collecting vehicle (6) comprises a duct system (15) through which the exhaust gas of the vehicle (2) can be routed.

21. Harvesting machine (1) according to any one of Claims 13 to 20, wherein the delivery duct (7) and/or the collecting container (8) are heat-insulated.

22. Harvesting machine (1) according to any one of Claims 13 to 21, wherein the delivery duct (7) and/or the collecting container (8) are sealed.

## Revendications

1. Procédé de production de protéines de stress à partir de matières végétales, comprenant les étapes consistant à :
(a) récolter les végétaux au moyen d'une moissonneuse et obtenir ainsi de la matière végétale tondue,
(b) chauffer la matière végétale pour induire la production de protéines de stress dans cette matière végétale immédiatement avant et/ou pendant et/ou après l'étape (a), et
(c) isoler les protéines de stress à partir de la matière végétale.

2. Procédé selon la revendication 1, dans lequel, à l'étape (b), le chauffage après l'étape (a) se fait au moins en partie par un échauffement résultant de processus endo-biochimiques dans la matière végétale.

3. Procédé selon l'une des revendications précédentes, dans lequel le chauffage se fait à une température dans la plage d'environ 30 à environ 60°C.

4. Procédé selon la revendication 3, dans lequel le chauffage immédiatement avant l'étape (a) se fait à une température d'environ 55°C.

5. Procédé selon la revendication 3, dans lequel le chauffage pendant et/ou après l'étape (a) se fait à une température d'environ 35 à environ 45°C.

6. Procédé selon l'une des revendications précédentes, dans lequel le chauffage se fait pendant une durée allant jusqu'à 75 heures.

7. Procédé selon la revendication 6, dans lequel le chauffage immédiatement avant l'étape (a) se fait pendant une durée d'environ 1 seconde à environ 1 minute, en particulier pendant environ 30 secondes.

8. Procédé selon la revendication 6, dans lequel le chauffage après l'étape (a) se fait pendant une durée allant jusqu'à 75 heures.

9. Procédé selon l'une des revendications précédentes, dans lequel le chauffage se fait au moins en partie en utilisant la chaleur dégagée par le système d'entraînement de la moissonneuse.

10. Procédé selon l'une des revendications précédentes, dans lequel la matière végétale est broyée après l'étape (b).

11. Procédé selon l'une des revendications précédentes, dans lequel la matière végétale est compactée.

12. Moissonneuse (1) destinée à être utilisée dans le procédé selon l'une des revendications précédentes, comprenant un véhicule (2) propulsé par moteur et un dispositif de tonte (3) supporté sur le véhicule, dans laquelle il est prévu sur le dispositif de tonte (3) et/ou sur le véhicule (2) une source de chaleur (4, 5) dirigée sur les végétaux avant le dispositif de tonte et/ou dirigée sur la zone des outils de tonte.

13. Moissonneuse (1) destinée à être utilisée dans le procédé selon l'une des revendications 1 à 11, comprenant un véhicule (2) propulsé par moteur et un dispositif de tonte (3) supporté sur le véhicule, dans laquelle il est prévu un dispositif de collecte (6), mû par le véhicule (2) ou automoteur, avec une goulotte d'amenée (7) et un bac de collecte (8) destiné à recevoir et transporter la matière végétale, la goulotte d'amenée (7) et/ou le bac de collecte (8) étant réalisés de telle sorte qu'il se produise dans la matière végétale un échauffement exothermique résultant de processus endo-biochimiques.

14. Moissonneuse (1) réalisée conformément aux revendications 12 et 13.

15. Moissonneuse (1) selon l'une des revendications 12 ou 13, dans laquelle il est prévu une source de chaleur (9) affectée à la goulotte d'amenée (7) et/ou au bac de collecte (8).

16. Moissonneuse (1) selon l'une des revendications 12, 14 ou 15, dans laquelle la source de chaleur (4, 5) dirigée sur les végétaux avant le dispositif de tonte et/ou sur les outils de tonte est réalisée sous la forme d'un radiateur, d'une soufflante ou d'une source de rayonnement à micro-ondes (17).

17. Moissonneuse (1) selon l'une des revendications 12 à 15, dans laquelle la source de chaleur (4, 5, 9) peut emmagasiner la chaleur dégagée par le véhicule.

18. Moissonneuse (1) selon l'une des revendications 13 à 17, dans laquelle le véhicule de collecte (6) présente une goulotte d'amenée (7) et/ou un bac de collecte (8) pour la matière végétale qui sont réalisés de telle sorte que la matière végétale puisse y être compactée.

19. Moissonneuse (1) selon l'une des revendications 15 à 18, dans laquelle la source de chaleur (9) affectée au véhicule de collecte (6) est affectée à la goulotte d'amenée (7) et/ou au bac de collecte (8).

20. Moissonneuse (1) selon l'une des revendications 14 à 19, dans laquelle la source de chaleur (9) affectée au véhicule de collecte (6) présente un système de conduites (15) permettant de canaliser les gaz d'échappement du véhicule (2).

21. Moissonneuse (1) selon l'une des revendications 13 à 20, dans laquelle la goulotte d'amenée (7) et/ou le bac de collecte (8) sont isolés thermiquement.

22. Moissonneuse (1) selon l'une des revendications 13 à 21, dans laquelle la goulotte d'amenée (7) et/ou le bac de collecte (8) sont rendus étanches.
